# EUROPEAN PATENT APPLICATION

(11) **EP 4 516 298 A1**
(43) Date of publication of application: **05.03.2025**
(21) Application number: 23194817.5
(22) Date of filing: 01.09.2023
(51) Int. Cl.: A61K 31/05, A61P 31/22

(54) **STILBENE COMPOUNDS FOR USE IN THE TREATMENT AND PREVENTION OF INFECTIONS WITH CYTOMEGALOVIRUS (CMV)**

(71) Applicant: Szekeres, Thomas, 1010 Wien (AT); Jäger, Walter, 3021 Pressbaum (AT); Steininger, Christoph, 1180 Vienna (AT)
(72) Inventor: Szekeres, Thomas, 1010 Wien (AT); Jäger, Walter, 3021 Pressbaum (AT); Steininger, Christoph, 1180 Vienna (AT)
(74) Representative: SONN Patentanwälte GmbH & Co KG

(57) **Abstract**

The invention discloses 3,3', 4,4',5,5'-hexahydroxy-trans-stilbene or a pharmaceutically acceptable ester thereof, for use in the treatment and prevention of infections with cytomegalovirus (CMV) in a human subject, especially for inhibiting human CMV.

## Description

The present invention relates to the treatment and prevention of diseases caused by infectious herpes viruses, especially by cytomegalovirus (CMV).

Herpesviridae ("herpesviruses") is a family of DNA viruses that cause infections and certain diseases in animals, including humans. As of 2020, 115 species are recognized, all but one of which are in one of the three subfamilies. Herpesviruses can cause both latent and lytic infections.

The human herpesvirus (HHV) is a spherical enveloped virus with a diameter ranged from 120 to 260 nm, usually about 150 nm. The genetic material of HHV contains a double-stranded DNA genome that encodes 100-200 genes in the decahedral capsid. The herpesvirus replicates in its host cell nucleus, and its genes express in a highly coordinated transcriptional cascade as the following order: (1) immediate early genes, which code for regulatory proteins essential for viral proliferation and reactivation; (2) early genes, which code for enzymes essential for viral DNA replication; (3) late genes, which code for structural proteins essential for infectious viral particles (virions) assembly.

Nine herpesvirus types are known to primarily infect humans, at least five of which are widespread among most human populations, and which cause common diseases: herpes simplex 1 and 2 (HSV-1 and HSV-2, also known as HHV-1 and HHV-2; both of which can cause orolabial and genital herpes), varicella zoster (or HHV-3; the cause of chickenpox and shingles), Epstein-Barr (EBV or HHV-4; implicated in several diseases, including mononucleosis and some cancers), and human cytomegalovirus (HCMV or HHV-5) . More than 90% of adults have been infected with at least one of these, and a latent form of the virus remains in almost all humans who have been infected. Other human herpesviruses are human herpesvirus 6A and 6B (HHV-6A and HHV-6B), human herpesvirus 7 (HHV-7), and Ka-posi's sarcoma-associated herpesvirus (KSHV, also known as HHV-8). Human herpes viruses can infect host cells in virtually any organ of the human body.

The life cycle of HHV can be divided into two types: lytic pathway and lysogenic pathway. In the lytic pathway, the virion attaches to a host cell with a specific receptor to initiate the process of infection. After the viral outer membrane-coated glycoprotein binds to the host's cell membrane receptor, virions enter host cells by receptor-mediated endocytosis or membrane fusion. After the viral capsid is decomposed, the genomic DNA is released. The invading DNA manipulates the host enzyme and energy system to synthesize new viral DNA and proteins and then assembles to form new virions. During the process of HCMV replication, the terminase complex cleaves DNA to package the genome into the capsid to complete DNA maturation and viral assembly. Finally, the new virions release from original host cells to infect new hosts. However, in the lysogenic pathway, a few viral genes may transcribe latency-associated transcripts (LATs) in host cells. In this pattern, the virus can exist indefinitely without lysing host cells and cause no signs and symptoms in the host, resulting in long-term latency. The latency is referred to the state in which the virus is dormant, and the latent infection is a persistent infection in which the viral DNA is present in the host cell. In this life cycle, viruses still can be transmitted to others in special cases (e.g., organ transplantation, congenital infection), although the virions cannot be detected and no obvious symptoms are found. When the virus is stimulated or the host immune system is suppressed, the dormant viruses can be reactivated to produce large numbers of viral progeny to cause symptoms and diseases. There are two types of viral latency: proviral latency and episomal latency. In the provirus latency, the viral genomic DNA is incorporated in the host genome after the provirus enters the host cell, and the viral DNA replicate synchronously with the host DNA, such as human immunodeficiency virus (HIV). In the episomal latency, the viral DNA exists in the cytoplasm or nucleus of the host in a linear or lariat structure without integrating into the host genome. Thus, the virus still can utilize its own genetic materials, such as HHV. After the latent HHV is reactivated, the transcription of the LAT gene would change to the lytic gene to enhance viral DNA replication and viral proliferation, which often causes non-specific initial symptoms clinically, such as fever, headache, sore throat, general malaise, rash, etc. These symptoms may worsen to cause severe diseases of patients, and in some severe cases, even lead to death (reviewed in Chen et al., Med. Infect. Dis. 7 (2022), 439).

At least four of the human herpes viruses, herpes simplex virus type 1 (HSV-1), herpes simplex virus type 2 (HSV-2), cytomegalovirus (CMV), and varicella zoster virus (VZV) are known to infect and cause lesions in the eye of certain infected individuals. These four viruses are the leading cause of infectious blindness in the developed world.

Although they may be found throughout the body, human CMV (HCMV) infections are frequently associated with the salivary glands. HCMV infection is typically unnoticed in healthy people, but can be life-threatening for the immunocompromised, such as HIV-infected persons, organ transplant recipients, or newborn infants. Congenital cytomegalovirus infection can lead to significant morbidity and even death. After infection, HCMV remains latent within the body throughout life and can be reactivated at any time. Eventually, it may cause mucoepidermoid carcinoma and possibly other malignancies such as prostate cancer and breast cancer (all reviewed in Chen et al., Med. Infect. Dis. 7 (2022), 439; Panda et al., viruses 15 (2023), 1358).

HCMV is found in all geographic locations and all socioeconomic groups, and infects between 60% and 70% of adults in the first world and almost 100% in the third world. Of all herpes viruses, HCMV harbours the most genes dedicated to altering (evading) innate and adaptive host immunity and represents a lifelong burden of antigenic T cell surveillance and immune dysfunction. Commonly it is indicated by the presence of antibodies in the general population. HCMV is also the virus most frequently transmitted to a developing fetus. HCMV infection is more widespread in developing countries and in communities with lower socioeconomic status and represents the most significant viral cause of birth defects in industrialized countries. Congenital HCMV is the leading infectious cause of deafness, learning disabilities, and intellectual disability in children (all reviewed in Chen et al., Med. Infect. Dis. 7 (2022), 439; Panda et al., viruses 15 (2023), 1358). CMV also "seems to have a large impact on immune parameters in later life and may contribute to increased morbidity and eventual mortality" (Caruso et al., Immun. & Ageing 6 (2009), 10).

There are no known cures for infections with human herpes viruses, i. e., methods of eliminating the virus from the body of the infected individual. In addition, there are very few methods for blocking the formation of infectious herpes virus particles and thereby reducing the frequency, severity, or duration of a herpes virus-induced infection and the likelihood of recurrence of infection in the latently-infected individual. Some antiviral drugs including ganciclovir, valganciclovir, cidofovir, and fos-carnet have been approved on the market and used clinically for the treatment of HCMV infections for years. The mode of action of these traditional drugs is to inhibit the viral DNA polymerase. Two novel antiviral drugs have been approved for clinical application for the treatment of HCMV infections in recent years, such as letermovir and maribavir acting on viral terminase complex or protein kinase. WO 00712534 A2 discloses a method of inhibiting the formation of infectious herpes virus particles, particularly infectious HSV particles, in a host cell by administering a hydroxylated stilbene, in particular resveratrol or a derivative thereof.

However, almost all traditional drugs must be injected intravenously and cannot be used in patients without side effects and causing other problems. That is, their efficacy is remains limited by toxicity, drug resistance, cross-resistance, and other issues; thus, the administration methods, effectiveness and safety need further improvement. Nucleic acid-based gene targeting approaches include strategies quite different from traditional antiviral drugs in the structure, mechanism, administration routes and delivery tools. They potentially provide effective strategies to treat diseases caused by HCMV infections by designing a specific DNA or RNA sequence to target essential genes for virus growth, such as EGSs-RNase, the CRISPRs/Cas9 nuclease system and TALENs. These unconventional antiviral approaches are probably promising in preventing HCMV infections or removing lytically and/or latently infected HCMV, however, these approaches are all in vitro and/or in vivo studies, and none of them have been advanced to clinical trials. Also cell therapies have been suggested (adoptive T-cell therapy for transplantation recipients and immunotherapy for glioblastoma (GBM)patients.

Thus, it is desirable to have additional methods for inhibiting the formation of infectious herpes virus particles and/or for preventing and treating herpesvirus infections or the diseases caused by such infections. Such method is useful for limiting the severity of a herpes virus infection within an infected individual and the likelihood of transmission of the herpes virus infection from the infected individual to a non-infected individual.

Therefore, the present invention provides 3,3',4,4',5,5'-hexahydroxy-trans-stilbene or a pharmaceutically acceptable ester thereof for use in the treatment and prevention of infections by or of diseases caused by infectious herpes viruses, preferably infectious human herpes viruses, especially by cytomegalovirus (CMV) .

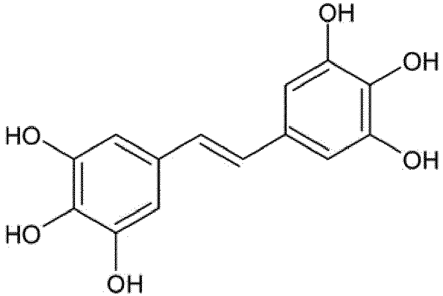

### 3,3',4,4',5,5'-Hexahydroxy-trans-stilbene (HHS)

3,3',4,4',5,5'-hexahydroxy-trans-stilbene and pharmaceutically acceptable esters thereof according to the present invention was identified in the course of the present invention to exhibit realistic and effective inhibitory activity towards herpes viruses, especially HCMV. The compounds according to the present invention can therefore effectively prevent or hinder herpes viruses from becoming pathogenic for human patients. Accordingly, the compounds according to the present invention are able to inhibit herpes viruses, especially HCMV, and/or to prevent or hinder herpes viruses, especially HCMV, from entering human cells at least to a certain extent to provide significant advantage for the patient to reduce the risk of developing diseases caused by these viruses. The compounds of the present invention are easily accessible, well tolerated and can successfully applied to humans without the need of invasive methods. Moreover, the compounds according to the present invention are regarded as "GRAS", i.e. they are "generally recognised as safe (i.a. under sections 201(s) and 409 of the US Federal Food, Drug, and Cosmetic Act.

WO 00712534 A2 suggests the use of hydroxylated stilbenes against herpes viruses, including resveratrol (3, 5, 4'-trihy-droxystilbene) and piceatannol (3,3',4,5'-tetrahydroxy-trans-stilbene), alleging both, the cis form or trans form being suitable for inhibiting the formation of herpes virus particles. While resveratrol was shown to suppress CMV replication in human fibroblasts (Izzo et al., Nutrients 13 (2021), 933), there are no clinical trials shown effectiveness for prevention or treatment efficiency of resveratrol in CMV infections in humans (Evers et al., Antivir. Res. 63 (2004), 85-95); Faith et al., Antivir. Res. 72 (2006), 242-251; Espinoza et al., PLoS ONE 7 (2012), e51306; Abba et al., Adv. Virol. (2015), 184241).

3,3',4,4',5,5'-hexahydroxy-trans-stilbene and pharmaceutically acceptable esters thereof have surprisingly turned out to be significantly more active in inhibiting herpes viruses, especially human CMV, than comparable polyphenolic substances suggested as being virus-inhibiting, such as resveratrol. Although resveratrol shows some inhibitory effect concerning human CMV, 3,3',4,4',5,5'-hexahydroxy-trans-stilbene and pharmaceutically acceptable esters thereof have significantly enhanced inhibitory effect over resveratrol.

3,3',4,4',5,5'-hexahydroxy-trans-stilbene and pharmaceutically acceptable esters thereof used according to the present invention are preferably inhibiting replication of herpes viruses, especially human CMV, and/or for preventing the cytopathic effect of an active virus replication of herpes viruses, especially human CMV, and/or preventing viral infections with herpes viruses, especially human CMV, through airborne channels.

Stilbene derivatives according to the present invention were previously disclosed e.g. in WO 02/50007 A2, WO 02/057219 A1, WO 2005/016860 A1, WO 2007/002973 A2 and WO2022/200086 A1.

These (poly-) hydroxylated phenols were described to have increased anti-cancer and anti-inflammatory effects in comparison to resveratrol due to the increased number of OH-groups in para position on polyhydroxylated stilbenes. Hexahydroxystilbene showed most effective anti-inflammatory and anti-cancer activity in vitro and anticancer effects in animal studies and was shown to inhibit HIV infection in vitro at a very early stage. These substances were also disclosed as regulators of T cells, neutrophils, macrophages and corresponding cytokines.

In CN 1736986 A, stilbene derivates were suggested as antiviral substances for SARS-CoV-1 wherein the compounds disclosed to have antiviral activity were pyridine-group containing compounds and 2,2' -OH or CH₃O- substituted compounds as well as 3, 3' methylbutenyl substituted compounds. Moreover, the nature of the compounds with actual anti-SARS activity were not disclosed.

3,3',4,4',5,5'-hexahydroxy-trans-stilbene and pharmaceutically acceptable esters thereof are excellent free radical scavengers, exhibiting a broad range of biochemical effects, such as inhibiting key enzymes of DNA synthesis or inflammation. In addition, some of them were shown to inhibit different virus infections through unspecific inhibition of virus entry or replication. The compounds were selected due to their chemical structure and availability in natural sources such as tea or fruits.

In the course of the present invention, 3,3',4,4',5,5'-hexahydroxy-trans-stilbene and pharmaceutically acceptable esters thereof were investigated regarding their inhibitory effects on virus infection using in vitro cell culture models.

In contrast to other suggestions in the prior art in which several polyphenolic substances were described to have potential anti-herpes virus, especially anti-human CMV, properties, it turned out with the present invention that polyphenolic substances highly vary in their effect against herpes virus, especially human CMV, and that only a few of these substances are effective.

These experiments performed in the course of the present invention showed that the synthetic resveratrol analogon according to the present invention and its pharmaceutically acceptable esters, exhibit inhibiting activity against herpes virus, especially human CMV, which significantly outperforms other natural polyphenolic compounds, even the few natural polyphenolic compounds which also turned out to have a real-world antiviral activity against herpes virus, especially human CMV (at least - for resveratrol - in human fibroblasts).

Preferred examples pharmaceutically acceptable esters of 3,3',4,4',5,5'-hexahydroxy-trans-stilbene for use according to the present invention include formyl ester, acetyl ester, propionyl ester, butyryl ester, fumaryl ester, sulfuryl ester, hydroxamate ester, and enanthate ester. Esterification of trans-stilbene compounds is well available to a person skilled in the art, e.g. by Tain et al. (Antiox. 10 (2021), 420), WO 2004/000302 A1, US 2017/0183290 A1, US 2014/134117 A1, and WO 2018/164662 A1. Specifically the pharmaceutically acceptable esters of 3,3',4,4',5,5'-hexahydroxy-trans-stilbene may be provided (e.g. as pro-drugs), for example as mono-, di-, tri-, tetra-, penta- or hexa-esters, preferably as mono-esters.

3,3',4,4',5,5'-hexahydroxy-trans-stilbene and pharmaceutically acceptable esters thereof used in the present invention are in principle known as pharmaceutical substances. Accordingly, the formulations already known and proven to be effective for delivery of these compounds to the human subject are also applicable for the present invention. The route of administration can be divided into enteral or parenteral, such as oral, sublingual, intramuscular, subcutaneous, nasal, oral mucosa, topical, especially a topical application to the eye, to the oral cavity, or to the lips; bronchial, pulmonary, skin, peritoneum or rectum, etc., preferably oral, sublingual or nasal, especially as an inhalable and/or aerosol composition or as a nasal or oral spray and/or as sublingual films or tablets and/or as lollipops. The compounds of the present invention or the pharmaceutical composition containing it can be administered in a unit dosage form. The dosage form for administration can be a liquid dosage form or a solid dosage form. For example, the liquid dosage form can be a true solution type, colloid type, microparticle dosage form, emulsion type, or suspension type. Examples of dosage forms include tablets; caplets; capsules, such as hard gelatin capsules and soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); sublingual films or tablets; lollipops; gels; liquid dosage forms suitable for oral or mucosal administration to a patient, including suspensions (e.g., aqueous or non-aqueous liquid suspensions, oil-in-water emulsions, or water-in-oil liquid emulsions), solutions, and elixirs. The compounds of the present invention can be made into ordinary preparations, and can also be slow-release agents, controlled-release agents, targeted preparations, and various particulate drug delivery systems, including inhalation systems (i.e. in the form of a kit comprising the pharmaceutical composition according to the present invention and an inhalator, such as the Diskhaler and other inhalators already used as inhalators for pharmaceutical compositions).

Preferably, the pharmaceutical composition is administered to an infected site during the prodromal stage of infection.

Therefore, the present invention relates to the use of a pharmaceutical preparation comprising 3,3',4,4',5,5'-hexahydroxy-trans-stilbene and pharmaceutically acceptable esters thereof according to the present invention with a pharmaceutically acceptable excipient. A pharmaceutically acceptable excipient may be any excipient known to be suitable and used for the compounds according to the present invention, especially a carrier or diluent. In order to make a unit dosage form, various carriers known in the art can be widely used.

Examples of carriers or diluents are, for example, absorbents, such as starch, dextrin, calcium sulfate, lactose, mannitol, sucrose, sodium chloride, glucose, urea, kaolin, microcrystalline cellulose, aluminium silicate, etc.; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone, etc.; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose, etc.; disintegration inhibitors, such as sucrose, glyceryl tristearate, cocoa butter, hydrogenated oil, etc.; absorption enhancers, such as quaternary ammonium salts, sodium lauryl sulfate, etc.; lubricants, such as talc, silicon dioxide, corn starch, stearic acid, boric acid, liquid paraffin, polyethylene glycol, etc.. Other carriers such as polyacrylic resins, liposomes, water-soluble carriers such as PEG4000 and PEG6000, PVP and so on. The tablets can also be further made into coated tablets, such as sugar-coated tablets, film-coated tablets, enteric-coated tablets, or double-layered and multi-layered tablets. For example, in order to make the administration unit into a pill, various carriers known in the art can be widely used. Examples of carriers are, for example, diluents and absorbents, such as glucose, lactose, starch, cocoa butter, hydrogenated vegetable oil, polyvinylpyrrolidone, kaolin, talc, etc.; binders, such as acacia, tragacanth, gelatin, Ethanol, honey, liquid sugar, rice paste or batter, etc.; disintegrants, such as agar powder, dried starch, alginate, sodium lauryl sulfonate, methyl cellulose, ethyl cellulose, etc.. For example, in order to make the dosing unit into a capsule, the compounds of the present invention as an active ingredient are mixed with the aforementioned various carriers, and the resulting mixture is placed in a hard gelatin capsule or a soft capsule. The active ingredient of the compound of the present invention can also be made into microcapsules, suspended in an aqueous medium to form a suspension, or filled into hard capsules or made into injections, inhalations or aerosols, for application. For example, the compounds of the present invention are prepared into an injection, an inhalation or aerosol preparation, such as a solution, a suspension solution, an emulsion, a lyophilized powder, and this preparation may be aqueous or non-aqueous.

The pharmaceutical preparation of the present invention may contain one and/or more pharmacodynamically acceptable excipients, such as carriers, diluents, binders, lubricants, preservatives, surfactants or dispersants. For example, the diluent can be selected from water, ethanol, polyethylene glycol, 1,3-propanediol, ethoxylated isostearyl alcohol, polyoxylated isostearyl alcohol, polyoxyethylene sorbitol fatty acid ester, etc.. In addition, in order to prepare an isotonic inhalation solution/suspension, aerosol, injection, an appropriate amount of sodium chloride, glucose or glycerin can be added to the injection preparation. In addition, conventional solubilizers, buffers, pH adjusters, etc. can also be added. These auxiliary materials are commonly used in this field. In addition, if necessary, colouring agents, preservatives, flavours, sweeteners, or other materials can also be added to the pharmaceutical preparation.

In order to achieve the purpose of medication and enhance the therapeutic effect, the drug or pharmaceutical composition of the present invention can be administered by any known administration method. The dosage of the compound or pharmaceutical composition of the present invention depends on many factors, such as the nature and severity of the disease to be prevented or treated, the gender, age, weight, personality and individual response of the patient or animal, the route of administration, the number of administrations and the purpose of treatment, so the therapeutic dose of the present invention can have a wide range of changes. Generally speaking, the dosage of the pharmacodynamic ingredient of the present invention is well known to those skilled in the art. According to the actual amount of the drug contained in the final formulation of the compound composition of the present invention, appropriate adjustments can be made to meet the requirements of the therapeutically effective dose, and the dosage of the compounds of the present invention can be completed, most preferably 0.1-20 mg/Kg body weight.

The above-mentioned dosage can be administered in a single dosage form or divided into several, for example, two, three or four dosage forms, which is limited by the clinical experience of the administering doctor and the dosage regimen including the use of other treatment means.

According to a preferred embodiment, 3,3',4,4',5,5'-hexahydroxy-trans-stilbene and pharmaceutically acceptable esters thereof according to the present invention are be formulated for delivery into the upper respiratory system. Exemplary formulations include nasal, bronchial, oral, and pulmonary formulations. However, the compounds according to the present invention may also be formulated for topical administration including a liquid, gel, wax, or paste. It is specifically preferred to formulate the present composition as an aerosol. The aerosol can be a liquid or powdered aerosol. In some embodiments, the composition contains one or more pharmaceutically acceptable excipients such as glycerol. The composition can contain 0.01%-20% w/v of the effective ingredient according to the present invention and 10% to 20% glycerol.

Pharmaceutical compositions and unit dosage forms of the present invention typically also include one or more pharmaceutically acceptable excipients, especially carriers or diluents. Advantages provided by specific compounds of the disclosure, such as increased solubility and/or enhanced flow, purity, or stability (e.g., hygroscopicity) characteristics can make them better suited for pharmaceutical formulation and/or administration to patients than the prior art.

Suitable excipients are well known to those skilled in the art of pharmacy or pharmaceutics. Whether a particular excipient is suitable for incorporation into a pharmaceutical composition or dosage form depends on a variety of factors well known in the art including the way in which the dosage form will be administered to a patient. For example, oral dosage forms such as tablets or capsules may contain excipients not suited for use in parenteral dosage forms. The suitability of a particular excipient may also depend on the specific active ingredients in the dosage form. For example, the decomposition of some active ingredients can be accelerated by some excipients such as lactose, or when exposed to water. Active ingredients that include primary or secondary amines are particularly susceptible to such accelerated decomposition.

Specifically preferred pharmaceutically acceptable excipients are antioxidants (reduction agents). Antioxidants commonly used in pharmaceutical compositions include citric acid and its salts (E330-E333), tartaric acid and its salts (E334-E337), phosphoric acid and its salts (E338-E343) and ethylenediaminetetraacetic acid (EDTA) and its salts (calcium disodium EDTA, E385), vitamins C, E, etc..

The disclosure further encompasses pharmaceutical compositions and dosage forms that include one or more compounds that reduce the rate by which an active ingredient will decompose. Examples of such compounds are stabilizers, such as antioxidants such as ascorbic acid, pH buffers, or salt buffers. In addition, pharmaceutical compositions or dosage forms of the disclosure may contain one or more solubility modulators, such as sodium chloride, sodium sulfate, sodium or potassium phosphate or organic acids. A specific solubility modulator is tartaric acid.

Like the amounts and types of excipients, the amounts and specific type of compounds according to the present invention in a dosage form may depend on factors such as the route by which it is to be administered to patients. Typical dosage forms of the compounds of the present invention contain the effective ingredient according to the present invention in an amount of from about 100 µg to about 10 g, preferably in an amount of from about 1 mg to about 1 g, more preferably in an amount of from 10 mg to 500 mg, even more preferably in an amount of from about 20 mg to about 300 mg. Preferred dosage forms contain the compounds of the present invention in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

Preferably, the pharmaceutical compositions may also include a carrier, for example a sugar alcohol such as but not limited to glycerol, mannitol, sorbitol, xylitol, and erythritol. In a specific embodiment, the sugar alcohol is glycerol.

Typical topical dosage forms include liquids, creams, lotions, ointments, gels waxes, pastes, sprays, aerosols, solutions, emulsions, and other forms know to one of skill in the art. In a preferred embodiment, the present compounds are delivered to oral, nasal, or bronchial tissue in a suitable topical dosage form.

For non-sprayable topical dosage forms, viscous to semi-solid or solid forms including a carrier or one or more excipients compatible with topical application and having a dynamic viscosity preferably greater than water are typically employed. Suitable formulations include solutions, suspensions, emulsions, creams, ointments, powders, gels, waxes, pastes, liniments, salves, and the like, which are, if desired, sterilized or mixed with auxiliary agents (e.g., preservatives, stabilizers, wetting agents, buffers, or salts) for influencing various properties, such as, for example, osmotic pressure.

Nasal spray drug products contain therapeutically active ingredients dissolved or suspended in solutions or mixtures of excipients in non-pressurized dispensers that deliver a spray containing a metered dose of the active ingredient. The dose can be metered by the spray pump or could have been pre-metered during manufacture. A nasal spray unit can be designed for unit dosing or can discharge up to several hundred metered sprays of formulation containing the drug substance. Nasal sprays are applied to the nasal cavity for local and/or systemic effects.

According to another preferred embodiment, the compounds according to the present invention are provided as inhalation solution and suspension drug products. Such products are typically aqueous-based formulations that contain therapeutically active ingredients and can also contain additional excipients. Aqueous-based oral inhalation solutions and suspension must be sterile. Inhalation solutions and suspensions are intended for delivery to the lungs by oral inhalation for local and/or systemic effects and are to be used with a specified nebulizer. An inhalation spray drug product consists of the formulation and the container closure system. The formulations are typically aqueous based and must be sterile. Inhalation sprays are intended for delivery to the lungs by oral inhalation for local and/or systemic effects. Other suitable topical dosage forms include sprayable aerosol preparations wherein the active ingredient, preferably in combination with a solid or liquid inert carrier, is packaged in a mixture with a pressurized volatile (e.g., a gaseous propellant, such as freon), or in a squeeze bottle. Examples of sprayable aerosol preparations include metered dose inhalers, dry powder inhalers, and nebulizers. Moisturizers or humectants can also be added to pharmaceutical compositions and dosage forms if desired.

It may also be advantageous to provide the pharmaceutical compositions according to the present invention in transdermal and mucosal dosage forms, such as ophthalmic solutions, patches, sprays, aerosols, creams, lotions, suppositories, ointments, gels, solutions, emulsions, suspensions, or other forms known to one of skill in the art. Dosage forms suitable for treating mucosal tissues within the oral cavity can be formulated as mouthwashes, as oral gels, or as buccal patches. Additional transdermal dosage forms include reservoir type or matrix type patches, which can be applied to the skin and worn for a specific period of time to permit the penetration of a desired amount of active ingredient. Suitable excipients (e.g., carriers and diluents) and other materials that can be used to provide transdermal and mucosal dosage forms are well known to those skilled in the pharmaceutical field, and depend on the particular tissue or organ to which a given pharmaceutical composition or dosage form will be applied. With that fact in mind, typical excipients include water, acetone, ethanol, ethylene glycol, propylene glycol, butane-1,3-diol, isopropyl myristate, isopropyl palmitate, mineral oil, and mixtures thereof, to form dosage forms that are non-toxic and pharmaceutically acceptable. Depending on the specific tissue to be treated, additional components may be used prior to, in conjunction with, or subsequent to treatment with the compounds according to the present invention. For example, penetration enhancers can be used to assist in delivering the active ingredients to or across the tissue. Suitable penetration enhancers include acetone; various alcohols such as ethanol, oleyl, a tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as TWEEN 80 (polysorbate 80) and SPAN 60 (sorbitan monostearate).

The pH of a pharmaceutical composition or dosage form, or of the tissue to which the pharmaceutical composition or dosage form is applied, may also be adjusted to improve delivery of the active ingredient(s). Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of the active ingredient(s) so as to improve delivery. In this regard, stearates can serve as a lipid vehicle for the formulation, as an emulsifying agent or surfactant, and as a delivery-enhancing or penetration-enhancing agent.

The compounds of the present invention can also be formulated as extended or delayed release formulations. Extended and delayed release formulations for various active ingredients are known in the art, for example by encapsulation.

The compounds of the present invention are present in the pharmaceutical composition in about 0.001% to about 50% w/v, typically from about 0.01% to about 0.1% w/v, more typically about 1 % to about 20% w/v. In a preferred embodiment, compounds of the present invention are present in about 0.01% to about 20% w/v. The pharmaceutically acceptable excipient and eventually other compounds or agents present in the pharmaceutical composition then add up to the 100%.

The compounds and compositions of the present invention are useful for the treatment of one of more symptoms of viral infection with herpes viruses, especially human CMV. Preferably, the pharmaceutical compositions according to the present invention are formulated for nasal or oral application, such as drops, applicators, or sprays. One embodiment provides the compositions according to the present invention for prophylactically or therapeutically treating patients which are infected by or are at risk of being infected by a herpes virus, especially human CMV, especially for use to prevent viral infections through airborne channels.

According to a further aspect, the present invention relates to the use 3,3',4,4',5,5'-hexahydroxy-trans-stilbene or a pharmaceutically acceptable ester thereof, according to the present invention for the manufacture of a pharmaceutical preparation according to the present invention for the treatment and prevention of diseases, disorders and symptoms associated with or caused by herpes virus infections in a human subject, especially for inhibiting a herpes virus, especially human CMV. Preferably, the use is for inhibiting replication of herpes viruses, especially human CMV, and/or for preventing the cytopathic effect of an active virus replication of herpes viruses, especially human CMV, and/or preventing viral infections with herpes viruses, especially human CMV, through airborne channels.

According to another aspect, the present invention relates to a method for the treatment and prevention of infections by or of diseases, disorders or symptoms caused by or associated with infectious herpes virus, especially human CMV, in a human subject, especially for inhibiting herpes virus, especially human CMV, wherein to a subject which is infected by or are at risk of being infected by a herpes virus, especially human CMV, an effective amount of 3,3',4,4',5,5'-hexahydroxy-trans-stilbene or a pharmaceutically acceptable ester thereof, according to the present invention or a pharmaceutical preparation according to the present invention is administered. This method is in particular suitable for inhibiting replication of herpes viruses, especially human CMV, and/or for preventing the cytopathic effect of an active virus replication of a herpes virus, especially human CMV, and/or preventing viral infections with herpes viruses, especially human CMV, through airborne channels in a human subject.

The present invention is further illustrated by the following examples and the drawing figures, yet without being restricted thereto.

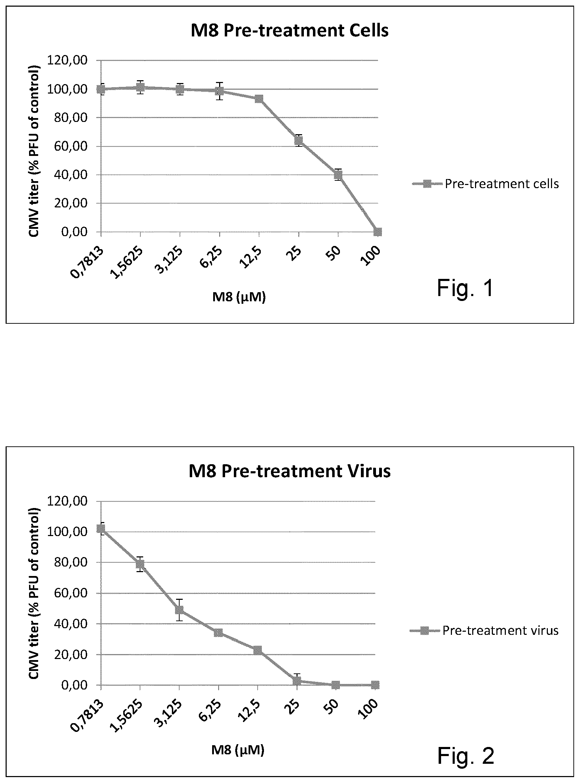

Fig. 1 shows the antiviral activity of 3,3',4,4',5,5'-hexahydroxy-trans-stilbene (compound "M8") when pretreating permissive cells. For the plaque reduction assay, HFF were seeded to 100 % confluency and then pre-treated with compound M8 at 8 different concentrations. HFF were subsequently infected with CMV strain AD169, washed and again incubated with two-fold dilutions of the compound. All experiments were done in triplicates (arrow bars show SEM). The x axis shows CMV titer (% PFU of control); the y axis shows concentration of M8 in µM.
Fig. 2 shows the antiviral activity of compound M8 when pretreating virus. For the plaque reduction assay, HFF were seeded to 100 % confluency. The virus preparation of CMV strain AD169 was pretreated before infection of cells with compound M8 at 8 different concentrations. HFF were subsequently infected with CMV strain AD169, washed and again incubated with two-fold dilutions of the compound. All experiments were done in triplicates (arrow bars show SEM). The x axis shows CMV titer (% PFU of control); the y axis shows concentration of M8 in µM.

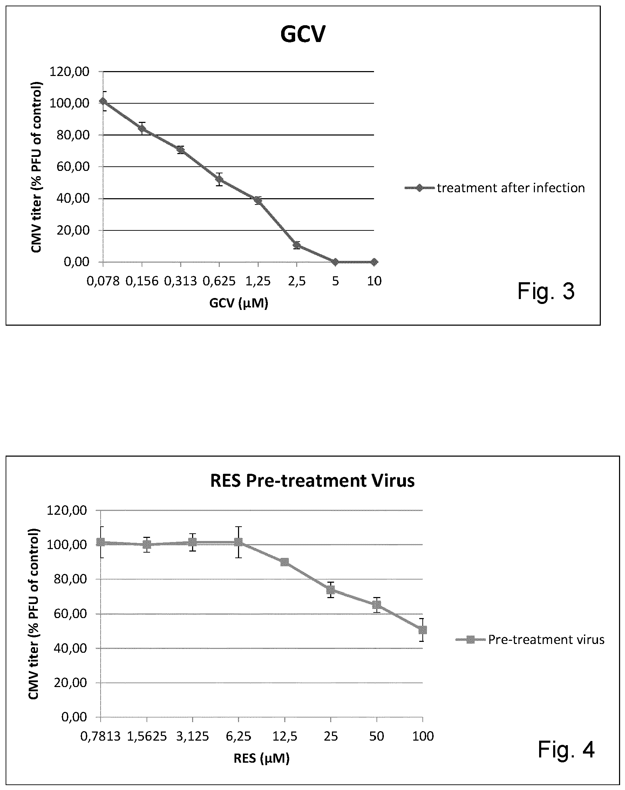

Fig. 3 shows the antiviral activity of compound Ganciclovir ("GCV"). For the plaque reduction assay HFF were seeded to 100 % confluency and subsequently infected with CMV strain AD169. Cells were washed thereafter and incubated with two-fold dilutions of the compound GCV. All experiments were done in triplicates (arrow bars show SEM). The x axis shows CMV titer (% PFU of control); the y axis shows concentration of GCV in µM.
Figs. 4 shows the antiviral activity of resveratrol (compound "RES") when pretreating permissive cells. For the plaque reduction assay HFF were seeded to 100 % confluency and then pre-treated with compound RES at 8 different concentrations. HFF were subsequently infected with CMV strain AD169, washed and again incubated with two-fold dilutions of the compound. All experiments were done in triplicates (arrow bars show SEM). The x axis shows CMV titer (% PFU of control); the y axis shows concentration of RES in µM.

### Examples:

In the present examples, human CMV inhibiting effects of 3,3',4,4',5,5'-hexahydroxy-trans-stilbene were investigated in comparison with resveratrol and ganciclovir, including functional plaque reduction assay of human CMV in a human foreskin fibroblast (HFF) cell culture assay with cells infected with human CMV.

### Materials and Methods

Cells were infected and incubated with different concentrations of all compounds.

### Functional plaque reduction assay - human CMV Infection

HCMV strain AD169 was prepared as described previously (Rieder et al., J. Gen. Virol. 98 (2017), 471-485). For virus production, HFF were grown to confluence and subsequently infected with HCMV AD169 at a multiplicity of infection (MOI) of 0.01. The viral inoculum was replaced by culture medium after 90 min. Upon visibility of a total cytopathic effect (CPE) (usually 8-10 days post-infection), the viral supernatant was harvested and stored at - 80°C. After thawing, cell debris was removed by centrifugation at 3,000 g for 20 min. Infectivity of the viral inoculum was then measured using a plaque assay as described elsewhere (Britt, Curr. Protoc. Microbiol. (2010), 14E.3). For functional plaque reduction assays, HFF were seeded into 24-well plates and either virus or cells pre-treated with vehicle (DMSO), 3,3',4,4',5,5'-hexahydroxy-trans-stilbene or resveratrol for 90 min at the indicated concentrations. The cells were then incubated with viral inoculum at 60 plaque forming units per well for 90 min, maintaining a constant concentration of DMSO, 3,3',4,4',5,5'-hexahydroxy-trans-stilbene or resveratrol through all phases of the experiment. Ganciclovir (Merck Millipore) was added at the indicated concentrations after removal of the inoculum. The cells were then overlaid with growth medium containing 0.5% low-melting point agarose. After solidification of the agarose layer, the plates were incubated for 10 days at 37°C in a CO₂ incubator, then fixed with 2% formaldehyde and analysed for number of plaques after staining with 1:5 diluted Mayer's Hemalaun. Plaque numbers were normalized to the mean of DMSO-treated control samples. IC50 values were calculated with Graphpad Prism (dose-response inhibition).

### Substances

### Substances

(3,3',4,4',5,5'-hexahydroxy-trans-stilbene, resveratrol and ganciclovir were dissolved in DMSO (Sigma) to substance stock concentrations of 5 mM or 1 mM, respectively. Further 1:2 dilutions were made to gain 2,5 mM and 0,5 mM stocks from certain substances. For the final assay concentration, a 1:100 dilution of the substance stocks in MEM 2 % per well was made. Substance stocks were prepared freshly prior to every assay.

### Results

To investigate whether compound M8 or its metabolites inhibit the replication of CMV, we performed plaque reduction assays in HFF treated with decreasing concentrations of M8, RES and GCV (Fig. 1-4). In these experiments, all three compounds showed a significant inhibition of CMV replication.

In cells pretreated with compound M8, some inhibition of CMV could be noted with a concentration of 12.5 µM, a complete inhibition at 100 µM and an IC50 at ~37.5 µM (Fig. 1). In the case of pretreatment of virus, inhibition by compound M8 was even more pronounced (Fig. 2). We noticed inhibition at a concentration of compound M8 at 6.25 µM and maximum inhibition at a concentration of 25 µM with an IC50 of ~3 µM. A concentration of M8 > 100 µM was found to be toxic to the HFF with inhibition of cellular metabolism.

In comparison, compound resveratrol (RES) also showed inhibition of CMV, as previously described, but not as pronounced as compound M8 (Fig. 4). We noticed inhibition at a concentration of compound RES at 6.25 µM and an IC50 of ~100 µM. Resveratrol was found to be also toxic to HFF at concentrations >100 µM.

Ganciclovir (GCV) was used as a reference compound with well-known inhibition of CMV in vitro and in vivo. GCV was used for treatment of HFF after CMV infection, as recommended by the company. As expected from the literature and our own previous studies, GCV showed an initial inhibitory effect at 0.156 µM and a complete inhibition at a concentration ≥5 µM.

In view of this disclosure and the examples, the following preferred embodiments are disclosed with the present specification:
1. 3,3',4,4',5,5'-hexahydroxy-trans-stilbene or a pharmaceutically acceptable ester hereof for use in the treatment and prevention of infections by or of diseases, disorders or symptoms caused by or associated with infectious herpes viruses, preferably by a herpes virus selected from the group consisting of HSV-1, HSV-2, CMV and VZV, especially by cytomegalovirus (CMV).
2. 3,3',4,4',5,5'-hexahydroxy-trans-stilbene or a pharmaceutically acceptable ester thereof for inhibiting formation of herpes virus particles, especially CMV particles, in a host cell.
3. 3,3',4,4',5,5'-hexahydroxy-trans-stilbene or a pharmaceutically acceptable ester thereof for blocking replication of a herpes virus, especially CMV, in a cell infected with the herpes virus.
4. 3,3',4,4',5,5'-hexahydroxy-trans-stilbene or a pharmaceutically acceptable ester thereof for use according to any one of embodiments 1 to 3, wherein 3,3',4,4',5,5'-hexahydroxy-trans-stilbene or a pharmaceutically acceptable ester thereof is administered prior to or within 6 hours of infection of the host cell with a herpes virus.
5. 3,3',4,4',5,5'-hexahydroxy-trans-stilbene or a pharmaceutically acceptable ester thereof for use according to any one of embodiments 1 to 4, wherein the pharmaceutically acceptable ester thereof is selected from formyl ester, acetyl ester, propionyl ester, butyryl ester, fumaryl ester, sulfuryl ester, hydroxamate ester, and enanthate ester, preferably a mono-, di-, tri-, tetra-, penta- or hexa-ester of any one of these esters, especially a mono-ester thereof.
6. 3,3',4,4',5,5'-hexahydroxy-trans-stilbene or a pharmaceutically acceptable ester thereof for use according to any one of embodiments 1 to 5, wherein 3,3',4,4',5,5'-hexahydroxy-trans-stilbene or a pharmaceutically acceptable ester thereof is included in a pharmaceutical composition which further comprises a topical carrier and which is administered is by topical administration to the skin.
7. 3,3',4,4',5,5'-hexahydroxy-trans-stilbene or a pharmaceutically acceptable ester thereof for use according to any one of embodiments 1 to 6, wherein inhibiting herpes virus is inhibiting replication of herpes virus and/or for preventing the cytopathic effect of an active virus replication of herpes virus and/or preventing viral infections with herpes virus through airborne channels.
8. A pharmaceutical preparation comprising or consisting of 3,3',4,4',5,5'-hexahydroxy-trans-stilbene or a pharmaceutically acceptable ester thereof according to any one of embodiments 1 to 7 as active ingredient and a pharmaceutically acceptable excipient for use in the treatment and prevention of infections by or of diseases caused by infectious herpes viruses, especially by cytomegalovirus (CMV).
9. A pharmaceutical preparation for use according to embodiment 8, wherein the preparation is for oral, sublingual, intramuscular, subcutaneous, nasal, oral mucosa, topical, especially a topical application to the eye, to the oral cavity, or to the lips; bronchial, pulmonary, skin, peritoneum or rectum administration, preferably for oral, sub-lingual or nasal administration, especially wherein the preparation is provided as an inhalable and/or aerosol composition or as a nasal or oral spray and/or as sublingual films or tablets and/or as lollipops.
10. A pharmaceutical preparation for use according to embodiment 8 or 9, wherein the pharmaceutical composition is administered to an infected site during the prodromal stage of infection.
11. A pharmaceutical preparation for use according to any one of embodiments 8 to 10, wherein the pharmaceutical is applied at or proximate a known site of infection or a site which is suspected of being infected.
12. A pharmaceutical preparation for use according to any one of embodiments 8 to 11, for reducing the symptoms of a herpes virus infection, especially an infection with CMV.
13. A pharmaceutical preparation for use according to any one of embodiment 8 to 12, wherein the pharmaceutically acceptable excipient is selected from diluents, such as water, acetone, ethanol, polyethylene glycol, polypropylene glycol, 1,3-propanediol, butane-1,3-diol, isopropyl myristate, isopropyl palmitate, mineral oil, ethoxylated isostearyl alcohol, polyoxylated isostearyl alcohol, polyoxyethylene sorbitol fatty acid ester; carrier, such as glycerol, mannitol, sorbitol, xylitol, and erythritol absorbents, such as starch, dextrin, calcium sulfate, lactose, mannitol, sucrose, sodium chloride, glucose, urea, kaolin, microcrystalline cellulose, aluminium silicate; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose; disintegration inhibitors, such as sucrose, glyceryl tristearate, cocoa butter, hydrogenated oil; absorption enhancers, such as quaternary ammonium salts, sodium lauryl sulfate; lubricants, such as talc, silicon dioxide, corn starch, stearic acid, boric acid, liquid paraffin, polyethylene glycol; polyacrylic resins, liposomes, water-soluble carriers such as PEG4000 and PEG6000, PVP; antioxidants; pH buffers and/or salt buffers; solubility modulators; penetration enhancers; antioxidants, such as citric acid and its salts, tartaric acid and its salts, phosphoric acid and its salts, and ethylenediaminetetraacetic acid (EDTA) and its salts, vitamins C, E; or mixtures thereof.
14. A pharmaceutical preparation for use according to any one of embodiments 8 to 13, wherein the preparation is an inhalation or aerosol preparation, especially an isotonic inhalation solution or suspension or a liquid or powdered aerosol.
15. A pharmaceutical preparation for use according to any one of embodiments 8 to 14, wherein the preparation contains 3,3',4,4',5,5'-hexahydroxy-trans-stilbene or a pharmaceutically acceptable ester thereof in an amount of from about 100 µg to about 10 g, preferably in an amount of from about 1 mg to about 1 g, more preferably in an amount of from 10 mg to 500 mg, even more preferably in an amount of from about 20 mg to about 300 mg.
16. A pharmaceutical preparation for use according to any one of embodiments 8 to 15, wherein the preparation contains 3,3',4,4',5,5'-hexahydroxy-trans-stilbene or a pharmaceutically acceptable ester thereof in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.
17. A pharmaceutical preparation for use according to any one of embodiments 8 to 16, wherein the preparation is contained in a metered dose inhaler, in a dry powder inhaler, or in a nebulizer.
18. A pharmaceutical preparation for use according to any one of embodiments 8 to 17, wherein the preparation contains 3,3',4,4',5,5'-hexahydroxy-trans-stilbene or a pharmaceutically acceptable ester thereof in 0.001% to 50% w/v, preferably in 0.01% to 20% w/v, more preferred in 0.1% to 20% w/v, especially in 1 % to 20% w/v.
19. Use of 3,3',4,4',5,5'-hexahydroxy-trans-stilbene or a pharmaceutically acceptable ester thereof according to any one of embodiments 1 to 7 for the manufacture of a pharmaceutical preparation according to any one of embodiments 8 to 18 for the treatment and prevention of diseases caused by herpes virus infections in a human subject, especially by human CMV.
20. Use according to embodiment 19 for inhibiting replication of a herpes virus, especially human CMV, and/or for preventing the cytopathic effect of an active virus replication of a herpes virus, especially human CMV and/or preventing viral infections with a herpes virus, especially CMV, through airborne channels.
21. Method for the treatment and prevention of diseases caused by a herpes virus, especially by CMV, in a human subject, especially for inhibiting a herpes virus, especially human CMV, wherein to a subject which is infected by or are at risk of being infected by a herpes virus, especially CMV, an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18 is administered.
22. Method for inhibiting replication of a herpes virus, especially human CMV, and/or for preventing the cytopathic effect of an active virus replication of a herpes virus, especially human CMV, and/or preventing viral infections with a herpes virus, especially human CMV, through airborne channels in a human subject, wherein to a subject which is infected by or are at risk of being infected by a herpes virus, especially human CMV, an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18 is administered.
23. A kit comprising
   (1) a container comprising an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18, suitable for inhalation; and
   (2) an inhalator.

## Claims

1. 3,3',4,4',5,5'-hexahydroxy-trans-stilbene or a pharmaceutically acceptable ester thereof, for use in the treatment and prevention of infections with cytomegalovirus (CMV) in a human subject, especially for inhibiting human CMV.

2. 3,3',4,4',5,5'-hexahydroxy-trans-stilbene or a pharmaceutically acceptable ester thereof, for use according to claim 1, wherein inhibiting CMV is inhibiting replication of CMV and/or for preventing the cytopathic effect of an active virus replication of CMV and/or preventing viral infections with CMV through airborne channels.

3. A pharmaceutical preparation comprising or consisting of 3,3',4,4',5,5'-hexahydroxy-trans-stilbene or a pharmaceutically acceptable ester thereof, for use according to claim 1 or 2 as active ingredient and a pharmaceutically acceptable excipient.

4. A pharmaceutical preparation for use according to claim 3, wherein the preparation is for oral, sublingual, intramuscular, subcutaneous, nasal, oral mucosa, bronchial, pulmonary, skin, peritoneum or rectum administration, preferably for oral, sub-lingual or nasal administration.

5. A pharmaceutical preparation for use according to claim 3 or 4, wherein the preparation is provided as an inhalable and/or aerosol composition or as a nasal or oral spray and/or as sublingual films or tablets and/or as lollipops.

6. A pharmaceutical preparation for use according to any one of claims 3 to 5, wherein the pharmaceutically acceptable excipient is selected from diluents, such as water, acetone, ethanol, polyethylene glycol, polypropylene glycol, 1,3-propanediol, butane-1,3-diol, isopropyl myristate, isopropyl palmitate, mineral oil, ethoxylated isostearyl alcohol, polyoxylated isostearyl alcohol, polyoxyethylene sorbitol fatty acid ester; carrier, such as glycerol, mannitol, sorbitol, xylitol, and erythritol absorbents, such as starch, dextrin, calcium sulfate, lactose, mannitol, sucrose, sodium chloride, glucose, urea, kaolin, microcrystalline cellulose, aluminium silicate; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose; disintegration inhibitors, such as sucrose, glyceryl tristearate, cocoa butter, hydrogenated oil; absorption enhancers, such as quaternary ammonium salts, sodium lauryl sulfate; lubricants, such as talc, silicon dioxide, corn starch, stearic acid, boric acid, liquid paraffin, polyethylene glycol; polyacrylic resins, liposomes, water-soluble carriers such as PEG4000 and PEG6000, PVP; antioxidants; pH buffers and/or salt buffers; solubility modulators; penetration enhancers; antioxidants, such as citric acid and its salts, tartaric acid and its salts, phosphoric acid and its salts, and ethylenediaminetetraacetic acid (EDTA) and its salts, vitamins C, E; or mixtures thereof.

7. A pharmaceutical preparation for use according to any one of claims 3 to 6, wherein the preparation is an inhalation or aerosol preparation, especially an isotonic inhalation solution or suspension or a liquid or powdered aerosol.

8. A pharmaceutical preparation for use according to any one of claims 3 to 7, wherein the preparation contains 3,3',4,4', 5,5'-hexahydroxy-trans-stilbene or a pharmaceutically acceptable ester in an amount of from about 100 µg to about 10 g, preferably in an amount of from about 1 mg to about 1 g, more preferably in an amount of from 10 mg to 500 mg, even more preferably in an amount of from about 20 mg to about 300 mg.

9. A pharmaceutical preparation for use according to any one of claims 3 to 8, wherein the preparation contains 3,3',4,4',5,5'-hexahydroxy-trans-stilbene or a pharmaceutically acceptable ester in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

10. A pharmaceutical preparation for use according to any one of claims 3 to 9, wherein the preparation is contained in a metered dose inhaler, in a dry powder inhaler, or in a nebulizer.

11. A pharmaceutical preparation for use according to any one of claims 3 to 9, wherein the preparation contains 3,3',4,4',5,5'-hexahydroxy-trans-stilbene or a pharmaceutically acceptable ester in 0.001% to 50% w/v, preferably in 0.01% to 20% w/v, more preferred in 0.1% to 20% w/v, especially in 1 % to 20% w/v.

12. Use of 3,3',4,4',5,5'-hexahydroxy-trans-stilbene or a pharmaceutically acceptable ester thereof according to claim 1 or 2 for the manufacture of a pharmaceutical preparation according to any one of claims 3 to 11 for the treatment and prevention of a CMV infection or a disease caused by a CMV infection a human subject, especially for the treatment and prevention of a mononucleosis caused by a CMV infection.

13. Use according to claim 12 for inhibiting replication of human CMV and/or for preventing the cytopathic effect of an active virus replication of human CMV and/or preventing viral infections with human CMV through airborne channels.

14. Method for the treatment and prevention of human CMV in a human subject, especially for inhibiting human CMV, wherein to a subject which is infected by or are at risk of being infected by human CMV an effective amount of 3,3',4,4',5,5'-hexahydroxy-trans-stilbene or a pharmaceutically acceptable ester thereof according to claim 1 or 2 or a pharmaceutical preparation according to any one of claims 3 to 11 is administered.

15. Method for inhibiting replication of cytomegalovirus (CMV) in a human subject, especially for inhibiting human CMV and/or for preventing the cytopathic effect of an active virus replication of CMV and/or preventing viral infections with CMV through airborne channels in a human subject, wherein to a subject which is infected by or are at risk of being infected by CMV an effective amount of 3,3',4,4',5,5'-hexahydroxy-trans-stilbene or a pharmaceutically acceptable ester thereof according to claim 1 or 2 or a pharmaceutical preparation according to any one of claims 3 to 11 is administered.
